# EUROPEAN PATENT SPECIFICATION

(11) **EP 3 662 973 B1**
(45) Date of publication and mention of the grant of the patent: **02.11.2022**
(21) Application number: 19158135.4
(22) Date of filing: 19.02.2019
(51) Int. Cl.: A61N 5/10

(54) **CALIBRATION OF HYPOXIC REGION FOCUSED RADIOTHERAPY TREATMENT PLANS**
KALIBRIERUNG VON AUF HYPOXISCHER REGION FOKUSSIERTEN STRAHLENTHERAPIEBEHANDLUNGSPLÄNEN
ÉTALONNAGE DE PLANS DE TRAITEMENT DE RADIOTHÉRAPIE FOCALISÉ SUR UNE RÉGION HYPOXIQUE

(30) Priority: 05.12.2018 US 201816210590
(43) Date of publication of application: 10.06.2020
(73) Proprietor: Nguyen, Nam, P., Washington, DC 20001 (US)
(72) Inventor: Nguyen, Nam, P., Washington, DC 20001 (US)
(74) Representative: Held, Stephan

(56) References cited:
- WO-A1-2014/062830
- US-A1- 2014 252 227

## Description

### FIELD OF INVENTION

The present invention relates to calibration of radiotherapy methods. More particularly, it relates to calibration of radiotherapy methods for hypoxic region focused tumor treatment as well as devices adapted to implement such methods.

### BACKGROUND OF THE INVENTION

### Tumor Hypoxia:

Tumor hypoxia refers to the condition in which cells within a tumor are deprived of oxygen. This condition can occur when a rapidly growing tumor lacks a functional vasculature system and thus has inadequate blood supply to regions in the interior of the tumor. Hypoxic regions within a tumor can be visualized using imaging techniques such as PET. Such non-invasive imaging techniques allow for identification of the boundaries of the hypoxic region or regions.

### Phantom Calibration of Radiotherapy Treatment Plans:

Radiotherapy plans are designed to provide sufficient dose levels to target tissues and limit the dose levels to surrounding organs at risk. Given the importance of proper radiation dosing, the treatment plans are routinely calibrated using a phantom before use in a patient. Irradiation of a phantom using the proposed treatment plan allows for a measurement of the actual radiation levels which are delivered using the plan. These actual radiation levels can be compared to the calculated radiation levels and the difference can be used to determine if the treatment plan requires revision.

### Radiotherapy Dose Limits:

Radiotherapy is a proven modality for cancer cure like surgery for tumors of all sites. The probability to destroy a cancer locally is proportional to the radiation dose delivered to the cancer site. Most often, an effectiveness of radiotherapy is limited by the radiation does that can safely be delivered to a normal organ adjacent to the tumor. Serious complications may occur if the normal organs received a radiation does that exceeds their tolerance to radiation. Paralysis (spinal cord injury), blindness (optic nerve injury), stroke (brain injury), bleeding (blood vessels injury), inflammation of lung (lungs injury) and bowels (bowels injury) may lead to death or seriously affect patient quality of life. These are well known complications of radiation treatment. Current methods of radiation treatment set a maximum limit for the radiation dose.

For example, in section 6.4.2.4 Radiation Therapy Oncology Group (RTOG) study number 0225: A Phase II Study of Intensity Modulated Radiation Therapy (IMRT) +/ Chemotherapy for Nasopharyngeal Cancer, it is specified that "No more than 20% of any PTV70 (the gross tumor volume with a 5 mm margin) will receive ≥ 110% of the prescribed dose." The rule limits the toxicity of treatment to avoid complications.

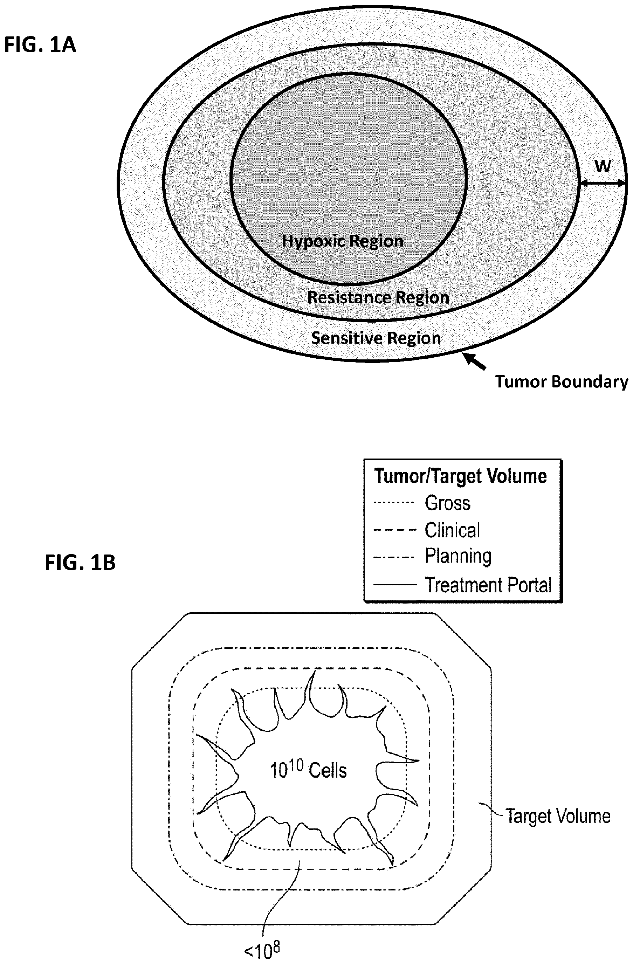

FIG. 1B shows schematic representation of "volumes" in radiation therapy in terms of Gross Target Volume, Clinical Target Volume, Planning Target Volume from Page 5, Chapter 1: The Discipline of Radiation Oncology, Book: Perez and Brady's Principles and Practice of Radiation Oncology, 5th Edition, published by Lippincott Williams & Wilkins with ISBN-10: 078176369X. This figure clearly shows that the planning target volume (PTV) is beyond the tumor boundary.

Table 1 shows the Memorial Sloan-Kettering Cancer Center (MSKCC) Clinical Dose Limits and Inverse Planning Algorithm Constraints for Primary Nasopharynx Tumors, excerpted from book "A practical guide to intensity-modulated radiation therapy" (Medical Physics Pub., 2003, ISBN: 1930524137), Chapter 10: IMRT for head and neck Cancer, Table 10.3, page 201. The table clearly regulates that the maximum dose is 105%.

**Table 1:**

| | | Inverse Planning Algorithm Constraint Template | | | |
|---|---|---|---|---|---|
| Structure | Clinical Dose Limits | Prescription Dose (%) | Maximum Dose (%) /Penalty | Minimum Dose (%) /Penalty | Dose (%)-%Volume Constraint/Penalty |
| PTVel | D₉₅ ≥50 Gy (95% of 54 Gy) | 54 Gy (77%) | 56.7 Gy (81%)/50 | 51.3 Gy (73%)/50 | NA |
| | Max. Dose | | | | |
| | ≤64.8 Gy (120% of 54 Gy) | | | | |
| PTV_{gr} | D₉₅ ≥70 Gy (100% of 70 Gy) | 70 Gy (100%) | 66.5 Gy (105%)/50 | 73.5 Gy (95%)/50 | NA |
| | Max. Dose | | | | |
| | ≤84 Gy (120% of 70 Gy) | | | | |
| Spinal Cord | Max. Dose | | 28 Gy | | NA |
| | ≤45 Gy | | (40%)/50 | | |
| Brainstem | Max. Dose | | 35 Gy | | NA |
| | ≤50 Gy | | (50%)/50 | | |
| Parotid | Mean Dose | | 68 Gy | | ≥21 Gy (30%) to |
| Gland | ≤26 Gy | | (98%)/50 | | ≤30% Volume/50 |
| Cochlea | Max. Dose | | 56 Gy | | NA |
| | ≤60 Gy | | (80%)/50 | | |

Table 2 shows the compliance criteria of radiation treatment in Radiation Therapy Oncology Group (RTOG) study number 0920: A Phase III Study of Postoperative Radiation Therapy (IMRT) +/- Cetuximab for Locally-Advanced Resected Head and Neck Cancer, section 6.7, page 27. The criteria list in Row 1 that any Radiation dose (RT) > 66Gy as a major variation should be avoided at any rate. The 66Gy corresponds to a 10% increase over PTV 60Gy.

**Table 2:**

| | **Per Protocol** | **Minor Variation** | **Major Variation** |
|---|---|---|---|
| Total RT dose to PTV60 (to 95% of PTV60) | 60-64 Gy | 58-60 or 64-66 Gy | < 58 or> 66 Gy |
| Minimum dose ("cold spot" within PTV60, not including portion of PTV near (<8 mm) skin) | 56-60 Gy | 54-56 Gy | < 54 Gy |
| Maximum dose ("hot spot") within PTV60* | < 70 Gy | 70-72 Gy | > 72 Gy |
| Maximum dose ("hot spot" outside of PTV60) | < 66 Gy | 66-70 Gy | > 70 Gy |
| Definition of CTV60 | Base on case review by study chair. | | |
| Definition of PTV60 | Base on case review by study chair. | | |
| Total RT dose to spinal cord PRV (0.03 cc) | < 48 Gy | 48-50 Gy | > 50 Gy |
| Total RT dose to spinal cord PRV (0.01 cc) | < 50 Gy | 50-52 Gy | > 52 Gy |
| Definition of Spinal cord PRV | Base on case review by study chair. | | |
| Overall RT treatment time | | | > 50 days (without a medically appropriate indication for delay) |
| Non-Medically Indicated Treatment Interruptions | 0-2 | 2-4 | > 4 |

| | | | |
|---|---|---|---|
| *Not including the region of PTV60 that falls within PTV66 (if applicable) | | | |

Table 3 shows the Critical Normal Structures in Radiation Therapy Oncology Group (RTOG) study number 0225: A Phase II Study of Intensity Modulated Radiation Therapy (IMRT) +/ Chemotherapy for Nasopharyngeal Cancer, section 6.4.3 Critical Normal Structures, page 7. The Critical Normal Structures discloses clearly that 60 Gy or 1 % of the PTV cannot exceed 65 Gy (which is close to 10% increase over PTV 60Gy radiation.

**Table 3:**

| | | |
|---|---|---|
| **6.4.3** | *Critical Normal Structures* | |
| | DVH's must be generated for all critical normal structures and the unspecified tissues. Dose | |
| | constraints to normal tissues will be as follows: | |
| | Brainstem, optic nerves, chiasm | 54 Gy or 1% of the PTV cannot exceed 60Gy |
| | Spinal Cord | 45 Gy or 1 cc *(if 1% is used, depends on length of the cord outlined)* of the PTV cannot exceed 50 Gy |
| | Mandible and T-M joint | 70 Gy or 1 cc of the PTV cannot exceed 75 Gy |
| | Temporal lobes | 60 Gy or 1 % of the PTV cannot exceed 65 Gy |
| | Unspecified tissue outside the targets: ≤ 100% of the dose prescribed to PTV₇₀. No more than 5% of the non-target tissue can receive greater than 70 Gy. **Participants are strongly encouraged to remain within these limits.** | |

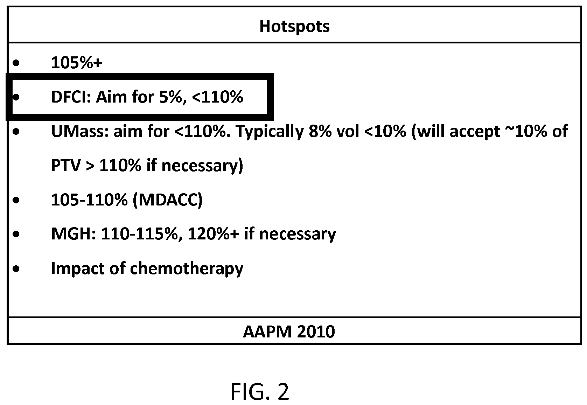

FIG. 2 shows the hotspot radiation regulation in a presentation (slide 13) of a research taken at Dana-Farber / Brigham & Women's Cancer Center and Harvard Medical School ("Variability in planning criteria and plan evaluation", Laurence Court, the American Association of Physicists in Medicine annual meeting 2010).The slide clearly shows the aiming for hotspots radiation is limited to <110% of the radiation dose.

However, a use of low radiation dose can be ineffective for curing cancer and a patient can die from uncontrolled tumor growth or from complications resulting from tumor destruction of normal organs. Thus, a clinician is often faced with a dilemma: either let the cancer kill the patient or expose the patient to serious injury from radiation complications. Therefore, there is a need for a balanced method for image-guided radiotherapy for providing higher dose for tumor tissue, such as hypoxia region of tumor, while avoiding excessive radiation to normal tissue.

US 2014/252227 A1 discloses a charged particle beam irradiation system for implementing an image guided radiotherapy, wherein the system comprises an irradiation unit configured to irradiate an irradiation target with a charged particle beam, a radiation resistance state measuring section configured to measure a radiation resistance state of the irradiation target; a region dividing section configured to divide the irradiation target into a plurality of radiation resistance regions based on a measurement result of the radiation resistance state measuring section; a radiation dose computing section configured to compute a planned value of a radiation dose of the charged particle beam for each of the plurality of radiation resistance regions divided by the region dividing section; and an irradiation planning section configured to create an irradiation plan of the charged particle beam with respect to the irradiation target based on a computation result of the radiation dose computing section.

WO 2014062830 discloses a similar image guided radiotherapy system.

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description and claims.

### SUMMARY OF THE INVENTION

The present invention features calibration of an image-guided radiotherapy treatment plan for a hypoxic tumor as well as devices or arrangements of devices adapted to implement such treatment plan. The treatment plans feature a hypoxic region focused (HRF) dose which may be greater than 120% of a predetermined prescribed radiation dose for the tumor. Additionally, the treatment plans may feature a resistance region focused (RRF) dose which is greater than the predetermined prescribed dose and less than the HRF dose.

One of the unique and inventive technical features of the present invention is that three-dimensional imaging is used to identify a hypoxic region of a tumor, and a resistance region is designated between the hypoxic region and a sensitive region. Without wishing to limit the invention to any theory or mechanism, it is believed that the technical feature of the present invention advantageously provides for calibration of HRF and RRF doses using irradiation of a phantom. None of the presently known prior references or work has the unique inventive technical feature of the present invention.

### BRIEF DESCRIPTION OF THE DRAWINGS

The features and advantages of the present invention will become apparent from a consideration of following detailed description presented in connection with the accompanying drawings in which:
FIG. 1A shows a schematic representation of a tumor with a sensitive region, a resistance region, and a hypoxic region, all within the tumor boundary. The average width of the sensitive region, W, is also shown.
FIG. 1B shows schematic representation of "volumes" in radiation therapy in terms of Gross Target Volume, Clinical Target Volume, Planning Target Volume.
FIG. 2 shows the hotspot radiation regulation in a presentation (slide 13) of a research taken at Dana-Farber / Brigham & Women's Cancer Center and Harvard Medical School.

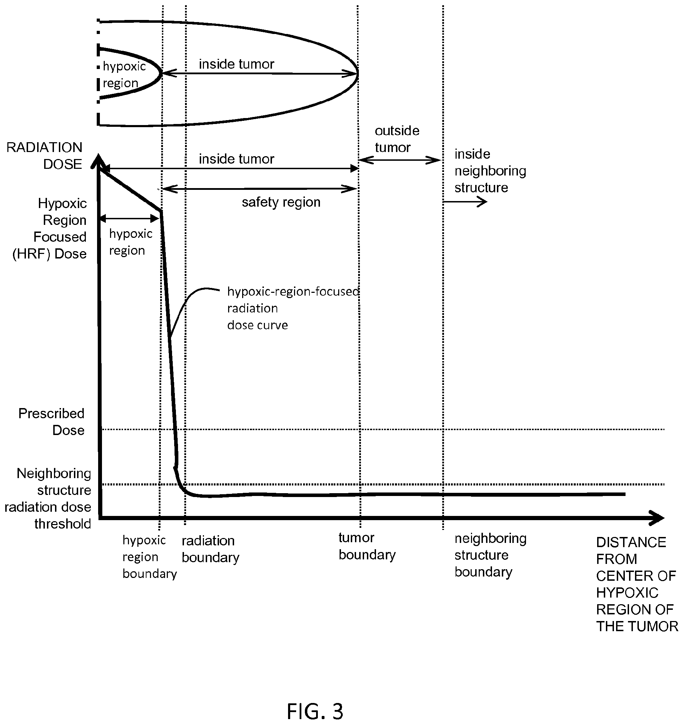

FIG. 3 is a schematic drawing of a tumor and the hypoxic region of the tumor. High HRF radiation dosage is applied in the hypoxic region. The figure is not to scale.

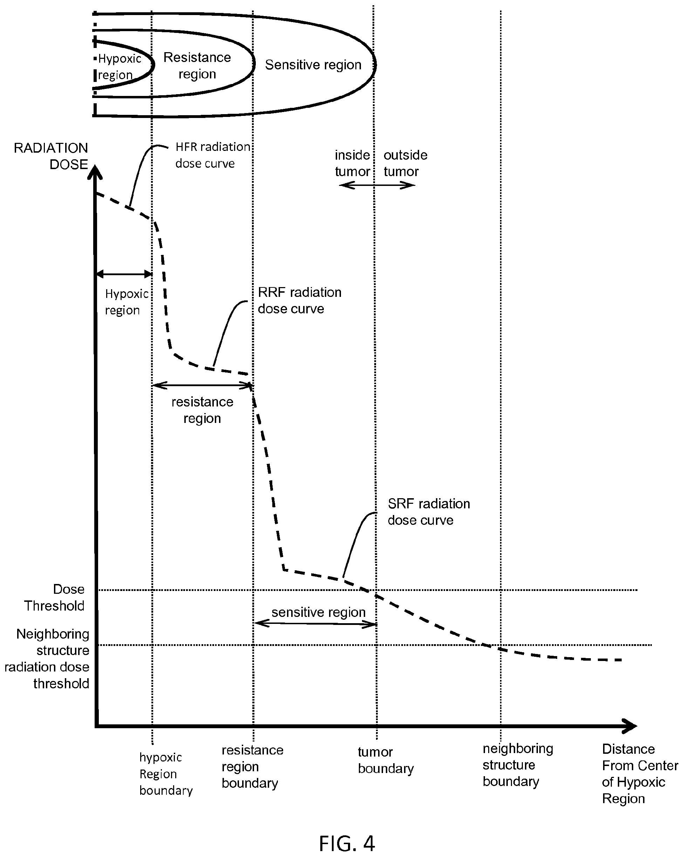

FIG. 4 is a schematic drawing of a tumor with a hypoxic region, a resistance region, and a sensitive region. A HRF radiation dose is applied in the hypoxic region, a RRF radiation dose is applied in the resistance region, and a SRF radiation dose is applied in the sensitive region of the tumor. At the tumor boundary, the dose is lower than a dose threshold. At the neighboring structure boundary, the dose is lower than a neighboring structure dose threshold. The figure is not to scale.

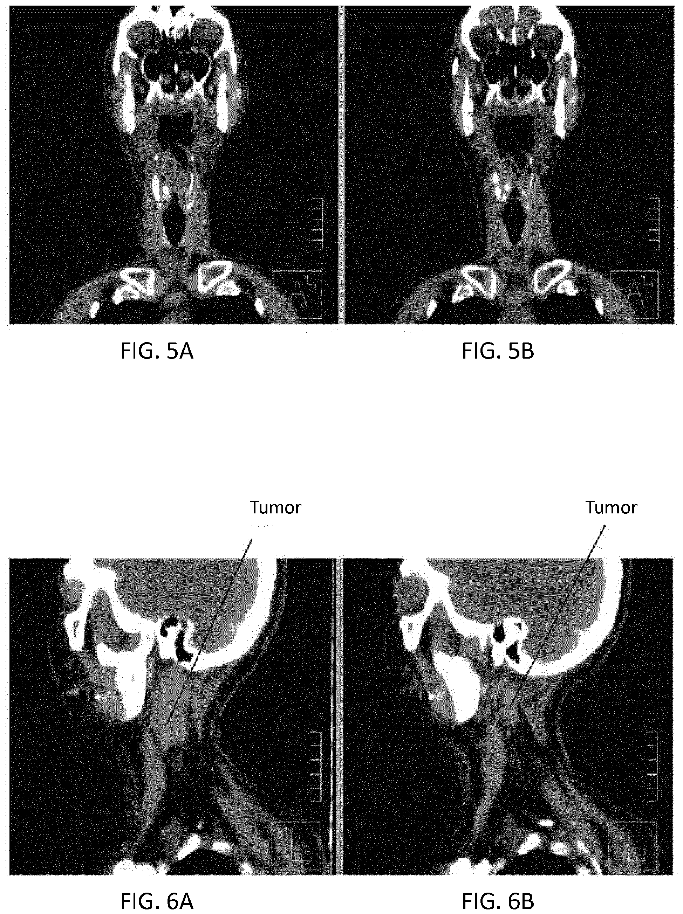

FIG. 5A and FIG. 5B are front views of clinical results showing tumor mass being removed due to high dosage radiation therapy by image guided boosted region technology.
FIGs. 6A and FIG. 6B are side views of clinical results showing tumor mass being removed due to high dosage radiation therapy by image guided boosted region technology.

### DETAILED DESCRIPTION OF THE INVENTION

As used herein, "hypoxic region" refers to a tumor region which has inadequate oxygen. As a non-limiting example, a hypoxic region may be a region in which is identified using a PET scan and a hypoxia sensitive biomarker. As used herein, "resistance region" refers to a tumor region which exhibits radiation resistant properties. As a non-limiting example, a resistance region may be a region in which surrounds the hypoxic area and in which the cancer cells are primarily dormant and non-dividing.

As used herein, the terms "prescribed radiation dose" or "prescribed dose" means the conventional dose established in the literature for cancer cure. The prescribed dose may be determined from external beam radiotherapy alone or radiotherapy combined with chemotherapy for locally advanced head and neck cancer. As non-limiting examples, the "prescribed dose" for Oropharyngeal cancer, Oral cavity cancer, Laryngeal cancer, Hypopharyngeal cancer is about 7000 cGy, at about 200 cGy per day.

As used herein "homogenous" refers to the quality of being substantially the same or similar throughout. For example, a homogenous dose may refer to a dose which is substantially the same throughout the homogenous volume. As another example a homogenous dose may refer to a dose which has no point throughout the homogenous volume where the dose differs from an average dose for the homogenous volume by more than about 1, 2, 3, 5, 10, or 15 percent. As used herein, the terms "boosted radiation dose" or "boosted dose" is defined as a dose that is at least 110% higher than the prescribed dose.

In one embodiment, the present invention features a method of calibrating a resistance region focused (RRF) dose in a radiotherapy treatment plan for a tumor using a phantom model. The inventive method comprises: obtaining a three-dimensional image of a tumor in a patient; identifying a peripheral boundary of the tumor using the three-dimensional image; identifying a hypoxic region within the tumor using the three-dimensional image; designating a region immediately inside the peripheral boundary as a sensitive region, the sensitive region having an average width W; designating a region between the hypoxic region and the sensitive region as a resistance region; generating a radiation treatment plan; irradiating a phantom following the treatment plan; measuring the irradiation in said phantom; testing the measured irradiation to the phantom region corresponding to the peripheral boundary to determine if the boosted RRF dose had increased irradiation at the peripheral boundary above a threshold value; and calibrating the RRF dose based on the determination of the irradiation to the phantom region corresponding to the peripheral boundary.

With regard to the above method, it is noted that the step of obtaining a three-dimensional image of a tumor in a patient is regularly a non-surgical and non-therapeutic step.

In the inventive method, the treatment plan, which is generated, comprises: a hypoxic region focused (HRF) dose, the HRF dose directed to the hypoxic region; a resistance region focused (RRF) dose, lower than the HRF dose but boosted above a spillover dose from the HRF dose, the RRF dose directed to the resistance region; and a sensitive region focused (SRF) dose, lower than the RRF dose, the SRF dose directed to the sensitive region.

In some embodiments, the tumor may be a radiation resistant tumor. In other embodiments, the three-dimensional image may be obtained using positron-emission tomography (PET). In still other embodiments, the HRF radiation dose or the RRF radiation dose may be greater than a prescribed treatment dose for the tumor. In yet other embodiments, a combined volume of the resistance region and the hypoxic region may be more than 20% of a total volume of the tumor. As a non-limiting example, the combined volume of the resistance region and the hypoxic region may be more than 25, 30, 35, 40, 45, 50, 60, 70, 80, 90, 95, or 99 percent of a total volume of the tumor.

Further disclosed, but not claimed, is an image-guided radiotherapy method for treatment of a tumor. This method may comprise: obtaining a three-dimensional image of a tumor in a patient; identifying a peripheral boundary of the tumor; identifying a hypoxic region within the tumor using the three-dimensional image; designating a region immediately inside the peripheral boundary as a sensitive region, the sensitive region having an average width W; designating a region between the hypoxic region and the sensitive region as a resistance region; designating and applying a hypoxic region focused (HRF) radiation dose to the hypoxic region; and designating and applying a resistance region focused (RRF) radiation dose to the resistance region. According to one embodiment, the HRF radiation dose may be greater than the RRF radiation dose. Therapeutic methods are not a part of the invention.

The method may further comprise designating and applying a sensitive region focused (SRF) radiation dose to the sensitive region. As an example, the SRF radiation dose may be lower than the RRF radiation dose. The radiation doses may be escalated in an iterative manner. As an example, a patient may be treated with a first radiation dose, imaged to determine the effect of the radiation dose on the tumor, and treated with a second radiation dose which is either larger or smaller than the first radiation dose, depending on if the effect of the radiation dose on the tumor.

The HRF radiation dose or the RRF radiation dose may be greater than a prescribed treatment dose for the tumor. The RRF dose may be greater than a spillover dose from the HRF dose. As an example, a spillover dose may decrease 10% in intensity with each additional mm of distance from the region to which the dose was directed. As another example, a spillover dose may decrease 1, 2, 3, 4, 5, 7, 15, 20, 30, 40, 50, 60, 70, 80, or 90 % in intensity with each additional mm of distance from the region to which the dose was directed. The tumor may be larger than about 3 cm in diameter. The tumor may alternatively be larger than about 0.5, 1, 1.5, 2, 2.5, 3.5, 4, 4.5, 5, 6, 7, 8, 9, or 10 cm in diameter.

Further disclosed, but not claimed, is an image-guided radiotherapy method for treatment of a tumor. This method may comprise: obtaining a three-dimensional image of a tumor; identifying a hypoxic region of the tumor, using the three-dimensional image; and designating and applying a hypoxic region focused (HRF) radiation dose to the hypoxic region. In a preferred embodiment, the HRF radiation dose may be at least 120 percent of a prescribed treatment dose for the tumor. The HRF radiation dose may be at least 105, 110, 115, 125, 130, 140, 150, 160, 170, 180, 190, 200, 250, 300, 400, 500 or 1000 percent of a prescribed treatment dose for the tumor. In some embodiments, the HRF radiation dose may be at least 30 Gy. In other embodiments, the HRF radiation dose may be at least 5, 10, 15, 20, 25, 35, 40, 45, 50, 60, 70, 80, 90, or 100 Gy.

The dose may reflect the dose delivered in one treatment session. The dose may reflect the total dose delivered over a number of treatment sessions. The method may further comprise designating a resistance region within the tumor and applying a resistance region focused (RRF) radiation dose to the resistance region. The RRF radiation dose may be lower than the HRF radiation dose.

In some embodiments of the inventive method, the three-dimensional image may be obtained using positron-emission tomography (PET). In other embodiments, the three-dimensional image may be obtained by magnetic resonance spectroscopy (MRS) or MRI. In a selected embodiment, the HRF radiation dose or the RRF radiation dose may greater than a prescribed treatment dose for the tumor. As a non-limiting example, the HRF radiation dose may be at least about 5, 10, 15, 20, 25, 30, 35, 40, 45, 50, 55, 60, 65, 70, 80, 90, or 100 Gy. As another non-limiting example, the RRF radiation dose may be at least about 5, 10, 15, 20, 25, 30, 35, 40, 45, 50, 55, 60, 65, 70, 80, 90, or 100 Gy. In some embodiments the HRF may be about 105, 110, 115, 120, 130, 140, 150, 160, 170, 180, 190, 200, 250, 300, 350, 400, 450, 500, 600, 700, 800, 900, or 1000 percent of the RRF dose. According to another embodiment, the method may further comprise designating a sensitive region focused (SRF) radiation dose to the sensitive region. In preferred embodiments, the SRF radiation dose may be lower than the RRF radiation dose. In some embodiments the RRF may be about 105, 110, 115, 120, 130, 140, 150, 160, 170, 180, 190, 200, 250, 300, 350, 400, 450, 500, 600, 700, 800, 900, or 1000 percent of the HRF dose.

In one embodiment, a volume of the hypoxic region may be more than 20% of a total volume of the tumor. In other embodiments, the volume of the hypoxic region may be more than 1, 2, 5, 10, 15, 25, 30, 40, 50, 60, 70, 80, 90 or 95% of a total volume of the tumor. In another embodiment, a combined volume of the resistance region and the hypoxic region may be more than 20% of a total volume of the tumor. In still other embodiments, the combined volume of the resistance region and the hypoxic region may be more than 1, 2, 5, 10, 15, 25, 30, 40, 50, 60, 70, 80, 90 or 95% of a total volume of the tumor. In an embodiment, the average width of the sensitive region, W, may be about 0.1, 0.5, 1, 2, 3, 4, 5, 6, 7, 8, 9, 10, 11, 12, 13, 14, 15, 16, 17, 18, 19, or 20 mm. In some embodiments the width of the sensitive region may be constant. In other embodiments, the width of the sensitive region may vary. According to one non-limiting example, a tumor having an average diameter of about 3 cm may have a sensitive region width of about 3-4 mm. According to another non-limiting example, a tumor having an average diameter of about 5 cm may have a sensitive region width of about 3-5 mm. According to yet another non-limiting example, a tumor having an average diameter of about 10 cm may have a sensitive region width of about 10 mm.

In another embodiment, the radiation doses may be escalated in an iterative manner. As a non-limiting example, an iterative treatment plan may double or triple the dose from one treatment to the next or reduce the dose from one treatment to the next. In yet another embodiment, the HRF dose may be homogenous over 95 percent by volume of the hypoxic region. In alternative embodiments, the HRF dose may be homogenous over 5, 10, 15, 20, 25, 30, 40, 50, 60, 70, 80, 90 or 99 percent by volume of the hypoxic region. Similarly, in some embodiments, the RRF dose may be homogenous over 95 percent by volume of the resistance region. In other alternative embodiments, the RRF dose may be homogenous over 5, 10, 15, 20, 25, 30, 40, 50, 60, 70, 80, 90 or 99 percent by volume of the resistance region.

The HRF radiation dose may be over 120% of a predetermined prescribed radiation dose for the tumor (110). As a non-limiting example, for radiation sensitive tumors, the predetermined prescribed radiation dose may range from about 180-200 centiGray per day and the HRF radiation dose may range from about 360 to 400 centiGray per day. As another non-limiting example, for radiation resistant tumors, the predetermined prescribed radiation dose may be about 250 centiGray per day and the HRF radiation dose may be about 500 centiGray per day.

Due to tail effect of an HRF radiation beam, a safety distance may affect the maximum HRF radiation dose. The HRF radiation dose may decrease at a rapid rate with increasing distance from the center of the hypoxic region such that sensitive region of the tumor (110) receives less than 40% of the HRF radiation dose.

According to some embodiments, the tumor may be a radiation resistant tumor. Examples of tumors that can be treated using this method include radiation sensitive tumors and radiation resistant tumors. Non-limiting examples of radiation sensitive tumors include: Squamous Carcinoma, Adeno Carcinoma, Small Cell Carcinoma, Lymphoma Carcinoma and Transitional Cell Carcinoma. Non-limiting examples of radiation resistant tumors include Melanoma and Renal cancer.

In yet another embodiment, the present invention features a device or arrangement of devices for implementing an image-guided radiotherapy method for treatment of a tumor, the device or arrangement of devices comprising:
a. means adapted for obtaining a three-dimensional image of a tumor in a patient;
b. means adapted for identifying a peripheral boundary of the tumor;
c. means adapted for identifying a hypoxic region within the tumor using the three-dimensional image;
d. means adapted for designating a region immediately inside the peripheral boundary as a sensitive region, the sensitive region having an average width W;
e. means adapted for designating a region between the hypoxic region and the sensitive region as a resistance region;
f. means adapted for designating and applying a hypoxic region focused (HRF) radiation dose to the hypoxic region; and
g. means adapted for designating and applying a resistance region focused (RRF) radiation dose to the resistance region, wherein the HRF radiation dose is greater than the RRF radiation dose;
h. means adapted for designating and applying a sensitive region focused (SRF) radiation dose to the sensitive region, wherein the SRF radiation dose is lower than the RRF radiation dose;
i. means adapted for irradiating a phantom, wherein the HRF radiation dose is applied to a hypoxic region of the phantom, the RRF radiation dose is applied to a resistance region of the phantom, and the SRF radiation dose is applied to the sensitive region;
j. means adapted for measuring the irradiation in said phantom;
k. means adapted for testing the measured irradiation to the phantom region corresponding to the peripheral boundary to determine if the boosted RRF dose had increased irradiation at the peripheral boundary above a threshold value; and
l. means adapted for calibrating the RRF dose based on the determination of the irradiation to the phantom region corresponding to the peripheral boundary.

Preferred embodiments of the above devices or arrangements of devices are described in dependent claims 8 to 14, and it is noted that all combinations of features in these claims with features from other dependent claims in the respective groups and their base claim are intended to be encompassed and described by the present specification.

With regard to the "means" mentioned above in a. to g. and a. to c., respectively, it is noted that the skilled practitioner is readily capable to select appropriate means in the form of devices suitable for the intended purpose, such as three dimensional imaging devices known in diagnostics, computers with analysis software to identify regions as mentioned above, and devices for applying radiation to designated parts of a body.

### Examples:

Example 1: The image-guided radiotherapy treatment plans in Table 4 may be calibrated using a phantom:

**Table 4: Example image-guided radiotherapy treatment plans**

| | Example 1 | Example 2 | Example 3 | Example 4 | Example 5 | Example 6 |
|---|---|---|---|---|---|---|
| Sensitive region dose | 0 Gy | 2 Gy | 2 Gy | 2 Gy | 2 Gy | 2 Gy |
| Resistance region dose | 5 Gy | 5 Gy | 10 Gy | 15 Gy | 20 Gy | 40 Gy |
| Hypoxic region dose | 10 Gy | 10 Gy | 20 Gy | 30 Gy | 40 Gy | 80 Gy |

Example 2: A patient may be treated according to any of the image-guided radiotherapy treatment plans in Table 4.

As used herein, the term "about" refers to plus or minus 10% of the referenced number.

The invention is defined in the claims. Other embodiments, examples, modifications and, in particular, therapeutic methods, are not a part of the invention.

Although there has been shown and described the preferred embodiment of the present invention, it will be readily apparent to those skilled in the art that modifications may be made thereto which do not exceed the scope of the appended claims. Therefore, the scope of the invention is only to be limited by the following claims. Reference numbers recited in the claims are exemplary and for ease of review by the patent office only, and are not limiting in any way. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting of", and as such the written description requirement for claiming one or more embodiments of the present invention using the phrase "consisting of" is met.

## Claims

1. A method of calibrating a resistance region focused, RRF, dose in a radiotherapy treatment plan for a tumor using a phantom model, the method comprising:
a. obtaining a three-dimensional image of a tumor in a patient;
b. identifying a peripheral boundary of the tumor using the three-dimensional image;
c. identifying a hypoxic region within the tumor using the three-dimensional image;
d. designating a region immediately inside the peripheral boundary as a sensitive region, the sensitive region having an average width W;
e. designating a region between the hypoxic region and the sensitive region
as a resistance region;
the method being **characterised by** the following steps:
f. generating a radiation treatment plan, the treatment plan comprising:
i. a hypoxic region focused, HRF, dose, the HRF dose directed to the hypoxic region;
ii. a resistance region focused, RRF, dose, lower than the HRF dose but boosted above a spillover dose from the HRF dose, the RRF dose directed to the resistance region; and
iii. a sensitive region focused, SRF, dose, lower than the RRF dose, the SRF dose directed to the sensitive region;
g. irradiating a phantom following the treatment plan;
h. measuring the irradiation in said phantom;
i. testing the measured irradiation to the phantom region corresponding to the peripheral boundary to determine if the boosted RRF dose had increased irradiation at the peripheral boundary above a threshold value; and
j. calibrating the RRF dose based on the determination of the irradiation to the phantom region corresponding to the peripheral boundary.

2. The method of claim 1, wherein the tumor is a radiation resistant tumor.

3. The method of claim 1, wherein the three-dimensional image is obtained using positron-emission tomography, PET.

4. The method of claim 1, wherein the HRF radiation dose is greater than a prescribed treatment dose for the tumor.

5. The method of claim 1, wherein the RRF radiation dose is greater than a prescribed treatment dose for the tumor.

6. The method of claim 1, wherein a combined volume of the resistance region and the hypoxic region is more than 20% of a total volume of the tumor.

7. A device or arrangement of devices for implementing an image-guided radiotherapy method for treatment of a tumor, the device or arrangement of devices comprising:
a. means adapted for obtaining a three-dimensional image of a tumor in a patient;
b. means adapted for identifying a peripheral boundary of the tumor;
c. means adapted for identifying a hypoxic region within the tumor using the three-dimensional image;
d. means adapted for designating a region immediately inside the peripheral boundary as a sensitive region, the sensitive region having an average width W;
e. means adapted for designating a region between the hypoxic region and the sensitive region as a resistance region;
f. means adapted for designating and applying a hypoxic region focused, HRF, radiation dose to the hypoxic region; and
g. means adapted for designating and applying a resistance region focused, RRF, radiation dose to the resistance region, wherein the HRF radiation dose is greater than the RRF radiation dose;
h. means adapted for designating and applying a sensitive region focused, SRF, radiation dose to the sensitive region, wherein the SRF radiation dose is lower than the RRF radiation dose;
i. means adapted for irradiating a phantom, wherein the HRF radiation dose is applied to a hypoxic region of the phantom, the RRF radiation dose is applied to a resistance region of the phantom, and the SRF radiation dose is applied to the sensitive region;
j. means adapted for measuring the irradiation in said phantom;
k. means adapted for testing the measured irradiation to the phantom region corresponding to the peripheral boundary to determine if the boosted RRF dose had increased irradiation at the peripheral boundary above a threshold value; and
l. means adapted for calibrating the RRF dose based on the determination of the irradiation to the phantom region corresponding to the peripheral boundary.

8. The device or arrangement of devices of claim 7, wherein the three-dimensional image is obtained using positron-emission tomography, PET.

9. The device or arrangement of devices of claim 7, wherein the means for applying radiation doses are adapted to escalate the radiation doses in an iterative manner.

10. The device or arrangement of devices of claim 7, wherein the HRF radiation dose is greater than a prescribed treatment dose for the tumor.

11. The device or arrangement of devices of claim 7, wherein the RRF radiation dose is greater than a prescribed treatment dose for the tumor.

12. The device or arrangement of devices of claim 7, wherein the RRF dose is greater than a spillover dose from the HRF dose.

13. The device or arrangement of devices of claim 7, wherein the tumor is larger than about 3 cm in diameter.

14. The device or arrangement of devices of claim 7, wherein a combined volume of the resistance region and the hypoxic region is more than 20% of a total volume of the tumor.

## Patentansprüche

1. Verfahren zur Kalibrierung einer auf einen Widerstandsbereich fokussierten (RRF) Dosis in einem Strahlentherapie-Behandlungsplan für einen Tumor unter Verwendung eines Phantom-Modells, wobei das Verfahren umfasst:
a. Erhalten eines dreidimensionalen Bildes eines Tumors in einem Patienten;
b. Identifizierung einer peripheren Begrenzung des Tumors unter Verwendung des dreidimensionalen Bildes;
c. Identifizierung eines hypoxischen Bereichs innerhalb des Tumors unter Verwendung des dreidimensionalen Bildes;
d. Bestimmung eines Bereichs unmittelbar innerhalb der peripheren Begrenzung als empfindlicher Bereich, wobei der empfindliche Bereich eine durchschnittliche Breite W aufweist;
e. Bestimmung eines Bereichs zwischen dem hypoxischen Bereich und dem empfindlichen Bereich als Widerstandsbereich;
wobei das Verfahren durch folgende Schritte gekennzeichnet ist:
f. Erzeugen eines Strahlungsbehandlungsplans, wobei der Behandlungsplan Folgendes umfasst:
i. eine auf den hypoxischen Bereich fokussierte (HRF) Dosis, wobei die HRF-Dosis auf den hypoxischen Bereich gerichtet ist;
ii. eine auf den Widerstandsbereich fokussierte (RRF) Dosis, die niedriger ist als die HRF-Dosis, aber über eine Spillover-Dosis der HRF-Dosis hinaus verstärkt ist, wobei die RRF-Dosis auf den Widerstandsbereich gerichtet ist; und
iii. eine auf den empfindlichen Bereich fokussierte (SRF) Dosis, die niedriger ist als die RRF-Dosis, wobei die SRF-Dosis auf den empfindlichen Bereich gerichtet ist;
g. Bestrahlung eines Phantoms gemäß dem Behandlungsplan;
h. Messung der Bestrahlung in dem Phantom;
i. Testen der gemessenen Bestrahlung des Phantombereichs, der der peripheren Begrenzung entspricht, um festzustellen, ob die verstärkte RRF-Dosis die Bestrahlung an der peripheren Begrenzung über einen Schwellenwert hinaus erhöht hat; und
j. Kalibrieren der RRF-Dosis auf der Grundlage der Bestimmung der Bestrahlung der Phantomregion, die der peripheren Begrenzung entspricht.

2. Verfahren nach Anspruch 1, wobei der Tumor ein strahlungsresistenter Tumor ist.

3. Verfahren nach Anspruch 1, wobei das dreidimensionale Bild mittels Positronen-Emissions-Tomographie (PET) erhalten wird.

4. Verfahren nach Anspruch 1, wobei die HRF-Strahlendosis größer ist als eine vorgeschriebene Behandlungsdosis für den Tumor.

5. Verfahren nach Anspruch 1, wobei die RRF-Strahlendosis größer ist als eine vorgeschriebene Behandlungsdosis für den Tumor.

6. Verfahren nach Anspruch 1, wobei das kombinierte Volumen der Resistenzregion und der hypoxischen Region mehr als 20% des Gesamtvolumens des Tumors beträgt.

7. Vorrichtung oder Anordnung von Vorrichtungen zur Durchführung eines bildgesteuerten Strahlentherapieverfahrens zur Behandlung eines Tumors, wobei die Vorrichtung oder Anordnung von Vorrichtungen umfasst
a. Mittel, die angepasst sind, ein dreidimensionales Bild eines Tumors in einem Patienten zu erhalten;
b. Mittel, die angepasst sind, eine periphere Begrenzung des Tumors zu identifizieren;
c. Mittel, die zur Identifizierung eines hypoxischen Bereichs innerhalb des Tumors unter Verwendung des dreidimensionalen Bildes angepasst sind;
d. Mittel, die angepasst sind, einen Bereich unmittelbar innerhalb der peripheren Begrenzung als einen empfindlichen Bereich zu bestimmen, wobei der empfindliche Bereich eine durchschnittliche Breite W hat;
e. Mittel, die angepasst sind, einen Bereich zwischen dem hypoxischen Bereich und dem empfindlichen Bereich als einen Widerstandsbereich zu bezeichnen;
f. Mittel, die angepasst sind, eine auf den hypoxischen Bereich fokussierte (HRF) Strahlendosis zu bestimmen und auf den hypoxischen Bereich anzuwenden; und
g. Mittel, die angepasst sind, eine auf den Widerstandsbereich fokussierte (RRF) Strahlungsdosis zu bestimmen und auf diesen anzuwenden, wobei die HRF-Strahlungsdosis größer ist als die RRF-Strahlungsdosis;
h. Mittel, die angepasst sind, eine auf den empfindlichen Bereich fokussierte (SRF) Strahlendosis zu bestimmen und auf den empfindlichen Bereich anzuwenden, wobei die SRF-Strahlendosis geringer ist als die RRF-Strahlendosis;
i. Mittel die zur Bestrahlung eines Phantoms angepasst sind, wobei die HRF-Strahlungsdosis auf einen hypoxischen Bereich des Phantoms, die RRF-Strahlungsdosis auf einen Widerstandsbereich des Phantoms und die SRF-Strahlungsdosis auf den empfindlichen Bereich angewendet wird;
j. Mittel angepasst zum Messen der Bestrahlung in dem Phantom;
k. Mittel angepasst zum Testen der gemessenen Bestrahlung des Phantombereichs, der der peripheren Grenze entspricht, um zu bestimmen, ob die verstärkte RRF-Dosis die Bestrahlung an der peripheren Grenze über einen Schwellenwert hinaus erhöht hat; und
l. Mittel angepasst zum Kalibrieren der RRF-Dosis auf der Grundlage der Bestimmung der Bestrahlung des Phantombereichs, der der peripheren Begrenzung entspricht.

8. Vorrichtung oder Anordnung von Vorrichtungen nach Anspruch 7, wobei das dreidimensionale Bild mittels Positronen-Emissions-Tomographie (PET) erhalten wird.

9. Vorrichtung oder Anordnung von Vorrichtungen nach Anspruch 7, wobei die Mittel zum Aufbringen von Strahlungsdosen so beschaffen sind, dass sie die Strahlungsdosen in einer iterativen Weise eskalieren.

10. Vorrichtung oder Anordnung von Vorrichtungen nach Anspruch 7, wobei die HRF-Strahlendosis größer ist als eine vorgeschriebene Behandlungsdosis für den Tumor.

11. Vorrichtung oder Anordnung von Vorrichtungen nach Anspruch 7, wobei die RRF-Strahlendosis größer ist als eine vorgeschriebene Behandlungsdosis für den Tumor.

12. Vorrichtung oder Anordnung von Vorrichtungen nach Anspruch 7, wobei die RRF-Dosis größer ist als eine Spillover-Dosis der HRF-Dosis.

13. Vorrichtung oder Anordnung von Vorrichtungen nach Anspruch 7, wobei der Tumor einen Durchmesser von mehr als etwa 3 cm hat.

14. Vorrichtung oder Anordnung von Vorrichtungen nach Anspruch 7, wobei das kombinierte Volumen der Widerstandsregion und der hypoxischen Region mehr als 20% des Gesamtvolumens des Tumors beträgt.

## Revendications

1. Procédé d'étalonnage d'une dose focalisée sur une région de résistance, RRF, dans un plan de traitement de radiothérapie pour une tumeur en utilisant un modèle fantôme, le procédé comprenant :
a. l'obtention d'une image 3D d'une tumeur chez un patient ;
b. l'identification d'une limite périphérique de la tumeur en utilisant l'image 3D ;
c. l'identification d'une région hypoxique à l'intérieur de la tumeur en utilisant l'image 3D ;
d. la désignation d'une région immédiatement à l'intérieur de la limite périphérique comme région sensible, la région sensible ayant une largeur moyenne W ;
e. la désignation d'une région entre la région hypoxique et la région sensible comme région de résistance ;
le procédé étant **caractérisé par** les étapes suivantes :
f. la génération d'un plan de traitement par rayonnement, le plan de traitement comprenant :
i. une dose focalisée sur une région hypoxique, HRF, la dose HRF dirigée sur la région hypoxique ;
ii. une dose focalisée sur une région de résistance, RRF, inférieure à la dose HRF mais potentialisée au-delà d'une dose de débordement de la dose HRF, la dose RRF dirigée sur la région de résistance ; et
iii. une dose focalisée sur une région sensible, SRF, inférieure à la dose RRF,
la dose SRF dirigée sur la région sensible ;
g. l'irradiation d'un fantôme selon le plan de traitement ;
h. la mesure de l'irradiation dans ledit fantôme ;
i. le test de l'irradiation mesurée de la région fantôme correspondant à la limite périphérique pour déterminer si la dose RRF potentialisée a accru l'irradiation au niveau de la limite périphérique au-delà d'une valeur seuil ; et
j. l'étalonnage de la dose RRF sur la base de la détermination de l'irradiation de la région fantôme correspondant à la limite périphérique.

2. Procédé selon la revendication 1, dans lequel la tumeur est une tumeur résistante aux rayons.

3. Procédé selon la revendication 1, dans lequel l'image 3D est obtenue par tomographie par émission de positons, PET.

4. Procédé selon la revendication 1, dans lequel la dose de rayonnement HRF est supérieure à une dose thérapeutique prescrite pour la tumeur.

5. Procédé selon la revendication 1, dans lequel la dose de rayonnement RRF est supérieure à une dose thérapeutique prescrite pour la tumeur.

6. Procédé selon la revendication 1, dans lequel un volume combiné de la région de résistance et de la région hypoxique est supérieur à 20 % d'un volume total de la tumeur.

7. Dispositif ou agencement de dispositifs pour mettre en œuvre un procédé de radiothérapie guidée par l'image pour le traitement d'une tumeur, le dispositif ou l'agencement de dispositifs comprenant :
a. un moyen adapté d'obtention d'une image 3D d'une tumeur chez un patient ;
b. un moyen adapté d'identification d'une limite périphérique de la tumeur ;
c. un moyen adapté d'identification d'une région hypoxique à l'intérieur de la tumeur en utilisant l'image 3D ;
d. un moyen adapté de désignation d'une région immédiatement à l'intérieur de la limite périphérique comme région sensible, la région sensible ayant une largeur moyenne W ;
e. un moyen adapté de désignation d'une région entre la région hypoxique et la région sensible comme région de résistance ;
f. un moyen adapté de désignation et d'application d'une dose de rayonnement focalisée sur une région hypoxique, HRF, sur la région hypoxique ; et
g. un moyen adapté de désignation et d'application d'une dose de rayonnement focalisée sur une région de résistance, RRF, sur la région de résistance, dans lequel la dose de rayonnement HRF est supérieure à la dose de rayonnement RRF ;
h. un moyen adapté de désignation et d'application d'une dose de rayonnement focalisée sur une région sensible, SRF, sur la région sensible, dans lequel la dose de rayonnement SRF est inférieure à la dose de rayonnement RRF ;
i. un moyen adapté d'irradiation d'un fantôme, dans lequel la dose de rayonnement HRF est appliquée à une région hypoxique du fantôme, la dose de rayonnement RRF est appliquée à une région de résistance du fantôme, et la dose de rayonnement SRF est appliquée à la région sensible ;
j. un moyen adapté de mesure de l'irradiation dans ledit fantôme ;
k. un moyen adapté de test de l'irradiation mesurée de la région fantôme correspondant à la limite périphérique pour déterminer si la dose RRF potentialisée a accru l'irradiation au niveau de la limite périphérique au-delà d'une valeur seuil ; et
l. un moyen adapté d'étalonnage de la dose RRF sur la base de la détermination de l'irradiation de la région fantôme correspondant à la limite périphérique.

8. Dispositif ou agencement de dispositifs selon la revendication 7, dans lequel l'image 3D est obtenue en utilisant la tomographie par émission de positons, PET.

9. Dispositif ou agencement de dispositifs selon la revendication 7, dans lequel le moyen d'application de doses de rayonnement est adapté pour augmenter les doses de rayonnement de manière itérative.

10. Dispositif ou agencement de dispositifs selon la revendication 7, dans lequel la dose de rayonnement HRF est supérieure à une dose thérapeutique prescrite pour la tumeur.

11. Dispositif ou agencement de dispositifs selon la revendication 7, dans lequel la dose de rayonnement RRF est supérieure à une dose thérapeutique prescrite pour la tumeur.

12. Dispositif ou agencement de dispositifs selon la revendication 7, dans lequel la dose RRF est supérieure à une dose de débordement de la dose HRF.

13. Dispositif ou agencement de dispositifs selon la revendication 7, dans lequel la tumeur a un diamètre supérieur à environ 3 cm.

14. Dispositif ou agencement de dispositifs selon la revendication 7, dans lequel un volume combiné de la région de résistance et de la région hypoxique est supérieur à 20 % d'un volume total de la tumeur.
